Europäisches Patentamt

European Patent Office

Office européen des brevets

(11) Publication number: **0 361 908**
**A2**

# EUROPEAN PATENT APPLICATION

(21) Application number: **89309863.2**

(22) Date of filing: **28.09.89**

(51) Int. Cl.5: **A61K 37/50 , A61K 47/00 , //C12P21/08**

(30) Priority: **28.09.88 US 250751**
**06.02.89 US 307119**

(43) Date of publication of application:
**04.04.90 Bulletin 90/14**

(84) Designated Contracting States:
**ES GR**

(71) Applicant: **IDEON CORPORATION**
**515 Galveston Drive**
**Redwood City California 94063(US)**

(72) Inventor: **Nedwin, Glenn E.**
**29 Fairway Place**
**Half Moon Bay California 94019(US)**
Inventor: **Köhn, James A.**
**1019 Thistle Court**
**Sunnyvale California 94086(US)**

(74) Representative: **Goldin, Douglas Michael et al**
**J.A. KEMP & CO. 14, South Square Gray's Inn**
**London WC1R 5EU(GB)**

(54) Combination enzyme immunotherapeutics.

(57) Target cells bearing a distinguishing surface antigen may be killed selectively, without harm to bystander cells, by administering myeloperoxidase, eosinophil peroxidase, thyroperoxidase, or salivary peroxidase, conjugated to a binding agent capable of specifically binding said surface antigen, and a hydrogen peroxide-producing enzyme conjugated to a second binding agent. The hydrogen peroxide-producing enzyme, in the presence of its substrate, generates hydrogen peroxide, which is utilized by the peroxidase to lethally halogenate the target cells.

## COMBINATION ENZYME IMMUNOTHERAPEUTICS

.

Technical Field

This invention relates to treatment of disease by administration of therapeutic agents. More specifically, this invention relates to the treatment of disease using enzymes or other proteins coupled to binding proteins specific for the cells or other organisms to be removed.

Background of the Invention

Since the development of monoclonal antibodies (MAbs), one goal of biotechnology has been the synthesis of immunotoxins, or "magic bullets": MAbs specific for a particular cell type (usually a malignant cell) coupled to an agent capable of a therapeutic effect. In general, a MAb is prepared having a specificity for an antigen present on the surface of the target cell, so that the MAb will bind to the target cell, and when internalized, carry its toxic conjugate inside. The toxic conjugate is most often ricin, or the ricin A subunit. The ricin B subunit binds to galactose residues ubiquitous on cell surfaces, while the A subunit enzymatically inactivates ribosomes. It has been estimated that a single molecule of recin can inactivate all of a cell's ribosomes. The invaded cell, unable to generate new protein, dies. As the B subunit is capable of binding to nearly any cell, it must be deactivated or excluded from any therapeutic use of ricin. For in vitro studies, ricin is typically used whole, with the B subunit saturated with galactose to reduce non-specific binding. In in vivo studies, the B subunit is usually removed, and a MAb-ricin A conjugate used. Although MAb conjugation increases the selectivity of ricin's cytotoxicity a great deal, serious residual toxicity still arises from non-specific binding of the MAb portion of the conjugate, e.g., binding due to Fc-receptors, antigenicity, etc.

Other toxic agents sometimes employed in immunotoxins include radioactive metal atoms, diphtheria toxin, and cancer chemotherapeutic agents. These immunotoxins exhibit residual toxicity similar to ricin-based immunotoxins, for the same reason.

S.J. Klebanoff (J Exp Med (1967) 126:1063-76) disclosed a method for killing bacteria in vitro using myeloperoxidase, iodide ions, and hydrogen peroxide. Hydrogen peroxide could be replaced by a combination of glucose and glucose oxidase, which in fact was more effective than $H_2O_2$. The bactericidal effect was apparently due to the formation of iodine-carbon bonds directly, rather than due to the formation of $I_2$ followed by iodination. Klebanoff also mentioned that iodide can be replaced with bromide or chloride. The assay conditions used required non-physiologic conditions.

Starkie (WO87/03205) disclosed a method for killing a cell or organism within a subject by linking an antibody specific for the cell or organism with a toxic enzyme, and administering the enzyme-linked antibody complex to the subject. Starkie exemplified glucose oxidase, which generates hydrogen peroxide and kills cells by oxidizing cell wall components. However, many cell types are resistant to hydrogen peroxide, e.g., due to endogenous anti-oxidation agents such as glutathione, and are refractory to such treatment.

T.J. Sullivan et al, Res Commun Chem Path Pharmacol (1973) 6:709-17 disclosed a method for treating Dirofilaria immitis by glucose oxidase (GO) conjugated to an anti-D. immitis antibody, followed by treatment with horseradish peroxidase (HRP) and phenylenediamine. The conjugated GO bound to the parasite surface, generating $H_2O_2$, which was in turn consumed by the free enzyme HRP while converting the phenylenediamine to cytotoxic radicals. Sullivan also disclosed radioiodination of the parasites using free lactoperoxidase and iodide in combination with the conjugated GO, but found no cytotoxic effect.

R.B. Lal et al, J Immunol Meth (1985) 79:307-18 disclosed a method for depleting selected cells of the immune system using conjugated enzymes. Lal screened free enzymes for cytolytic effect, determining that glucose oxidase and phospholipase C (PLC) were cytotoxic, but that xanthine oxidase, phospholipase $A_2$, and phospholipase D were ineffective, while choline oxidase was effective in the presence of 10 mM choline. Lal then conjugated GO and PLC to a murine monoclonal anti-rat kappa antibody, and targeted murine lymphocytes using rat anti-mouse MAbs (specific for lymphocyte antigens). Conjugated GO or PLC added at $2.5 \times 10^{-8}$ g/mL produced 25% and 28% killing, while the combination of conjugated GO with conjugated PLC produced 75% killing. Conjugated GO or PLC were able to kill 85-90% of targeted thymocytes at $250 \times 10^{-8}$ g/mL; however, conjugated GO was cytotoxic to non-targeted cells at $25 \times 10^{-8}$ g/mL.

J. Pene et al, Biochem Internat (1986) 13:233-43 disclosed that myeloma tumor cells can be killed in

2

vitro by treatment with tumor specific rabbit antisera (polyclonal), followed by incubation with glucose oxidase (GO) and lactoperoxidase (LPO) conjugated to anti-rabbit Ig MAbs, followed by incubation in glucose and NaI. The treatment utilizing all components (with NaI at 0.1 mM) was sufficient to cause release of 88% of $Cr^{51}$ in myeloma cells in vitro, whereas omission of any component caused $Cr^{51}$ release to drop to control values.

Disclosure of the Invention

One aspect of the invention is a method for killing target cells bearing a surface antigen characteristic of said target cells in a subject having such cells, which method comprises administering an effective amount of a peroxidase selected from myeloperoxidase, eosinophil peroxidase, thyroperoxidase, and salivary peroxidase, where said peroxidase is chemically conjugated to a first binding agent capable of specifically binding to the surface antigen, and a sufficient amount of a hydrogen peroxide-producing enzyme chemically conjugated to a second binding agent (which may be identical to the first binding agent) capable of specifically binding to a surface antigen. The administration may be in vivo, ex vivo, or in vitro. The presently preferred peroxidase is myeloperoxidase (MPO), especially human MPO (hMPO). Presently preferred hydrogen peroxide- producing enzymes are glucose oxidase, NADPH oxidase, amino acid oxidase, alcohol oxidase, xanthine oxidase, cholesterol oxidase, and choline oxidase. The binding agents are preferably antibodies, particularly monoclonal antibodies, but may additionally be receptor ligands, antibody or receptor fragments, lectins, and the like.

Another aspect of the invention comprises treating bone marrow ex vivo with an effective amount of peroxidase selected from myeloperoxidase, eosinophil peroxidase, thyroperoxidase, and salivary peroxidase, chemically conjugated to a first binding agent capable of specifically binding to a surface antigen, and a sufficient amount of a hydrogen peroxide-producing enzyme chemically conjugated to a second binding agent capable of specifically binding to a surface antigen. Presently preferred subclasses of the invention are the method where the target cells are normal lymphocytes (wherein the bone marrow is purged of immune cells to prevent graft vs. host syndrome), or where the target cells are cancer cells (wherein autologous bone marrow is purged prior to reintroduction into a subject being treated with cancer chemotherapy or radiotherapy). Another subclass is the method wherein the target cells are infected, and the surface antigen is a viral or microbial antigen expressed on the surface of the target cells.

Another aspect of the invention is a composition comprising an effective amount of peroxidase selected from myeloperoxidase, eosinophil peroxidase, thyroperoxidase, and salivary peroxidase, conjugated to a binding agent, an effective amount of a hydrogen peroxide-producing enzyme conjugated to a binding agent, and a pharmaceutically acceptable carrier.

Brief Description of the Drawing

Figure 1 graphically depicts the selective cytotoxicity of the method of the invention, as described in Example 2.

Figure 2 graphically depicts inhibition of viral plaque formation as described in Example 3.

Modes of Carrying Out The Invention

A. Definitions

The term "target cell" as used herein refers to any cell one desires to destroy which is characterized by a surface antigen distinguishing it from all non-target cells. A target cell is selected for destruction in this invention where such destruction will result in a therapeutic effect. Target cells include, without limitation, malignant or neoplastic cells, autoimmune lymphocytes, hyperproliferating cells, virally infected cells, bacteria, fungi, yeast, protozoa, viruses, and other parasites (one may also consider multi-cellular parasites within this definition). For example, in the case of cancer, removal of malignant and neoplastic cells effects remission. In autoimmune disease, it is desirable to remove those lymphocytes mediating an immune response against self components, e.g., T-cells cytotoxic to pancreatic islet cells in insulin-dependent diabetes mellitus. It may be useful to kill T-cells cytotoxic to a particular foreign MHC antigen, in the case of a non-histocompatible organ transplant. Viral infection could be checked by targeting cells bearing viral antigens on their surface.

The term "binding agent" as used herein refers to any ligand which is capable of binding to the desired surface antigen without substantial binding to non-target cells. The most typical binding agent is an antibody specific for the surface antigen in question. Antisera and monoclonal antibodies are now easily prepared which are highly specific for nearly any protein or carbohydrate antigen. Where the surface antigen constitutes a receptor for a specific ligand, the ligand may serve as the binding agent. For example, melanoma cells may carry a 1,25-dihydroxy vitamin D receptor: 1,25-dihydroxy vitamin D could be used as the binding agent in such cases. Other possible binding agents include, without limitation, antibody fragments such as Fab and F(ab')$_2$.

The term "surface antigen" as used herein refers to a protein, carbohydrate, lipid, or the like, which is characteristic of the target cell, and which is capable of specifically binding a binding agent. Suitable surface antigens may be tumor-specific proteins, such as oncofetoproteins, which are characteristic of tumors but are not expressed by ordinary cells. Viral antigens are also suitable, as they are typically expressed on the surface of infected cells. Where a specific immunosuppression is desired, e.g., to suppress graft rejection or an autoimmune reaction, the T-cell antigen receptor and/or B-cell surface-bound immunoglobulin may be used as the surface antigen. By selecting a binding agent which is selective for the particular receptor or immunoglobulin, one can delete only those T-cells or B-cells which participate in the graft rejection or autoimmune response. Where it is desired to purge bone marrow of all lymphocytes, e.g., to prevent graft vs. host disease, or to remove malignant lymphocytes for an autologous bone marrow transplant, one may use other lymphocyte-specific markers such as Thyl, T4 antigen, T8 antigen, the IL-2 receptor, IgG, IgM (constant region), and the like.

The terms "peroxidase" and "PO" as used herein refer to an enzyme selected from myeloperoxidase (MPO), eosinophil peroxidase (EPO), thyroperoxidase (TPO), and salivary peroxidase (SPO). The term "myeloperoxidase" refers to the enzyme typically isolated from myeloid cells (particularly neutrophils). The peroxidases used in the invention are capable of utilizing hydrogen peroxide as a substrate, and can oxidize halide ions. The oxidized halide product may be a hypohalous acid, a dihalogen molecule (such as $Cl_2$, $I_2$, etc.), or a reactive halo intermediate (for example a halogen radical). Hydroxide radicals may also be generated. In any event, the peroxidase will, in the presence of hydrogen peroxide, convert local halide ions (particularly iodide and chloride) into cytotoxic halogen species. Myeloperoxidase derived from humans (hMPO) is presently preferred, although MPO from other species may also be employed. Alternatively, one may use peroxidases derived using recombinant DNA techniques, including recombinant peroxidase derivatives which are altered from the native structure. For example, one may clone MPO, and express a truncated protein encoding only the active site of the enzyme, so long as the native peroxidase activity is substantially preserved. Alternatively, as MPO is an $(AB)_2$ type tetramer, consisting of two disulfide-linked AB dimers, one can reduce the central disulfide bonds to provide "hemi-myeloperoxidase" AB dimers retaining the full MPO specific activity, as described by P.C. Andrews et al, J Biol Chem (1981) 256:4211-18. The terms "peroxidase" and "PO" include peroxidases selected from myeloperoxidase, eosinophil peroxidase, thyroperoxidase, and salivary peroxidase obtained from humans, and from other species exhibiting at least 80% amino acid homology and cytotoxic halogen species generation activity with hPOs, and modified PO enzymes such as hemi-myeloperoxidase and recombinant POs exhibiting at least 30% of hPO's cytotoxic halogen species generation activity.

The terms "hydrogen peroxide-producing enzyme" and "HPPE" refer to an enzyme which, in the presence of its substrate, generates hydrogen peroxide. Exemplary HPPEs include, without limitation, glucose oxidase (GO), NADPH oxidase (particularly human NADPH oxidase), amino acid oxidase, alcohol oxidase, xanthine oxidase, cholesterol oxidase, choline oxidase, and the like. Preferably, the HPPE selected will utilize a substrate present endogenously in quantities sufficient for the HPPE to generate the amount of HOOH necessary for the peroxidase cytotoxic reaction. For example, the HPPE glucose oxidase generates enough HOOH in the presence of normal serum glucose for conjugated MPO to kill the target cell to which it is bound. If desired, some substrate concentrations may be regulated: for example, serum glucose levels may be modulated by administering carbohydrates or insulin. For other HPPEs, the substrate may be administered as necessary. For example, one may employ alcohol oxidase, and coadminister a quantity of ethanol sufficient to raise plasma alcohol to the necessary level. As with peroxidase, enzymes derived from humans are preferred, although enzymes form other animals will usually be acceptable (e.g., many useful HPPEs are derived from fungi). Additionally, one may use recombinant enzymes, including enzymes truncated or modified to comprise, for example, only the active site.

The terms "chemically conjugated" and "conjugated" as used herein mean that the enzyme and binding agent are connected by covalent bonds. This is accomplished by means known in the art as gener ally applied to labeling antibodies with enzymes (such as horse radish peroxidase) for ELISA assays, typically by using bifunctional linking reagents such as SPDP, MBS, SMCC and the like. Each molecule of binding agent may be linked to one or more molecules of enzyme. The enzyme and binding agent may alternatively be chemically conjugated by recombinant methods, i.e., by expression of the enzyme and binding agent as a single fusion protein. For example, to target T-cells using the interleukin-2 receptor, one could prepare an expression vector (e.g., a plasmid) having a gene encoding IL-2 (or the binding portion thereof) fused in frame with a gene encoding a peroxidase, such as myeloperoxidase or eosinophil peroxidase. Expression of this compound gene would provide an IL-2/MPO or IL-2/EPO fusion protein, having an IL-2 binding domain capable of binding to the T-cell IL-2 receptor, and an MPO or EPO domain capable of effecting the cytotoxic method of the invention.

Due to the number of variables, it is difficult to specify an exact "effective amount" of conjugated peroxidase. The effective amount, however, may be calculated or estimated empirically by one of ordinary skill in the art. This amount may be estimated considering the peroxidase turnover rate (rate of cytotoxic halogen species production), the number of target cells, the susceptibility of the target cells to the cytotoxic halogen, the rate of endocytosis by the target cell (which may remove the enzymes from their substrate sources), the concentration of substrate, the percentage of peroxidase inactivated by the conjugation process, etc. As a rough estimate, an effective amount may be taken as a number of peroxidase conjugates equal to the number of target cells, divided by the proportion of peroxidase conjugates inactivated during processing.

Similarly, it is difficult to specify a "sufficient amount" of HPPE, as the necessary amount depends on the turnover rate of the particular HPPE selected, the HOOH consumption rate of the peroxidase, etc. The sufficient amount may however be determined by routine experimentation, or by calculation. As a first approximation, one can use equal amounts of HPPE conjugates and peroxidase conjugates, where both enzymes have turnover rates of the same general magnitude. If the HPPE has a lower rate under the conditions of substrate concentration, pH, temperature, etc., used, then a correspondingly greater amount may be employed. The amount of substrate necessary may be calculated by similar means.

B. General Method

Peroxidases may be purchased from commercial sources, or may be purified from appropriate cells or cell lines. MPO may be obtained from myeloid cells or cell lines. The hMPO employed in the Examples below was derived from a human promyelocytic leukemia cell line designated HL-60, described by S.J. Collins et al, Nature (1977) 270:347-49, which is available from the American Type Culture Collection, Accession No. CCL240, and may be purified following the method of M. Yamada et al, Biochem (1981) 20:766-71.

Hydrogen peroxide-producing enzymes are known in the art, and may be purchased from commercial sources or purified from readily available sources using methods known in the art. For example, glucose oxidase obtained from Aspergillus niger may be purchased from Boehringer Mannheim.

Binding agents are most commonly antibodies. Polyclonal antisera may be obtained by hyperimmunizing a suitable mammal (e.g., rat, mouse, rabbit, goat) with the surface antigen, usually as an emulsion in complete Freund's adjuvant. The sera is obtained after 4-6 weeks by bleeding the animal, and purifying the blood by standard techniques. More preferred binding agents are monoclonal antibodies, which may be generated following the method of Kohler and Millstein, Nature (1975) 256:495-96. The binding agents may be the same for both enzymes, but are not required to be identical. The binding agents may in fact bind to different antigens, as long as the combination of both antigens is characteristic of the target cell population. For example, a population of T-cells may be depleted by treatment with anti-Thy1/GO + anti-Thy1/MPO; by anti-Thy1/GO + anti-IL-2 receptor/MPO; and by anti-Thy1/GO + anti-transferrin receptor/MPO. Although the transferrin receptor appears on many cell types, it appears in combination with the Thy1 antigen only on T-cells: thus, non-target cells are not subject to the cytotoxic effects of the method of the invention.

The MAbs (or other binding agents) may then be conjugated to the enzymes using techniques common for preparing enzyme-antibody conjugates for ELISA assays. For example, one may employ n-succinimidyl 3-(2-pyridyldithio)propionate (SPDP), 2-iminothiolane, succinimidyl 4-(N-maleimidomethyl)cyclohexane-1-carboxylate (SMCC), and the like, following the procedures described in J.M Lambert et al, J Biol Chem (1985) 260:12035-41.

The target cell is generally identified during diagnosis of a subject's condition. For example, a patient presenting with a palpable tumor may be subjected to biopsy to determine the presence of suitable surface antigens on tumor cells by screening against the wide variety of tumor cell-specific MAbs now available.

5

Many tumor types are already known to express certain characteristic antigens, for example lung small-cell carcinoma, B-cell lymphoma, and the like. Leukemic cells are often identified during standard microscopic assays, usually supplemented by MAb-based diagnostics. General immunosuppression, either systemic or ex vivo, may be effected by using MAbs specific for lymphocytes (e.g., OKT4, anti-IL-2 receptor, and the like). Viral antigens are well known for many viruses, and suitable MAbs are easily determined and obtained. A non-antibody binding agent may also be selected. For example, immunosuppression may be effected using IL-2 as the binding agent. Autoimmune disorders are particularly suited to such treatment as one may employ the "self" antigen in question, for example, pancreatic islet cell antigens may be used as the binding agent in insulin-dependent diabetes mellitus (the conjugated enzymes would then bind specifically to T-cells carrying antigen receptors autoreactive toward islet cells). Also, non-MHC matched grafts may be improved by using enzymes conjugated to the foreign (graft) MHC: T-cells capable of binding the foreign MHC would then be depleted without affecting other immune cells, thus effecting a limited, selective immunosuppression.

The conjugated enzymes may be assayed for activity in vitro to determine dosage, etc. The following assay employs 3-(4,5-dimethylthiazol-2-yl)-3,5-diphenyltetrazolium bromide (MTT), a dye which is metabolized by mitochondria, and is thus useful for indicating viable cells. Target cells are plated onto 96-well flat-bottom plates at about $1.5 \times 10^5$ cells/well, in a buffer containing $Ca^{++}$ and $Mg^{++}$, and 2% fetal bovine serum (FBS), but lacking enzyme substrates (e.g., halides, glucose, etc., depending on enzymes employed). Ionic strength is preferably adjusted with salts such as acetate, phosphate, and the like. Serial dilutions of the conjugated enzymes are then added to the plates in the same buffer, and are incubated for about 1 hour at $37°C$, under 5-7% $CO_2$. The plates are washed three times by spinning for 5 minutes at 1200 rpm, removing the liquid, and refilling with buffer. The enzyme substrate is then added (buffer containing 4.5 g/L glucose with 0.1 mM NaI), and the plates incubated for 2 hours at $37°C$ (5-7% $CO_2$) followed by washing three times as before. The cells are then resuspended in 100 uL of RPMI containing 5% FBS, 10 uL well MTT (5 mg/mL) added, and allowed to incubate for 4 hours. The plates are spun again, and liquid removed. DMSO (100%) is then added to each well (100 uL well), and absorbance read at 550 nm. The absorbance readings correlate with the number of viable cells. Other acceptable assay methods include such techniques as $^{51}Cr$ release, trypan dye exclusion, etc.

Once effective concentrations have been established, the conjugated enzymes are administered. For in vivo treatment of cancer, autoimmune disease, viral or other parasitic infection, hyperproliferative syndromes, and the like, the conjugated enzymes are administered in a suspension intravenously. For most hydrogen peroxide-producing enzymes, sufficient substrate will be present from endogenous sources. However, for some enzymes, for example alcohol oxidase, it may be desirable to increase the substrate concentration following the enzyme administration (e.g., by oral administration of ethanol). One may additionally administer small quantities of iodide, either in the form of an iodide salt or bound to a carrier protein such as thyroxine or triiodothyronine. However, endogenous chloride should provide sufficient halide for cytotoxicity.

Bone marrow is preferably treated ex vivo, e.g., for autologous bone marrow transplant, or for non-MHC matched bone marrow donation. Autologous transplantation is particularly useful for patients having cancer or blood-borne disease (e.g., chronic myelogenous leukemia, HIV-1, HIV-2, HTLV-I, HTLV-II, malaria, and the like) who may be treated with full body radiation. Bone marrow is removed from the patient, and purged of tumor cells or infected cells using the immunoconjugates of the invention. The method of the invention is particularly useful as it is highly specific, and does not damage hematopoietic stem cells in the bone marrow, as is the case with chemotherapeutic techniques and ricin conjugates. Once the bone marrow has been purged, the patient receives a full body dose of radiation, followed by reintroduction of his purged bone marrow. The replaced stem cells colonize the spleen and bone marrow and resume production of blood cells, thus avoiding much of the anemia and immunosuppression which would otherwise result. One may additionally use this technique to purge lymphocytes from bone marrow obtained from a donor having MHC (major histocompatibility) antigens different from the recipient. The donor bone marrow is extracted, and treated with enzymes conjugated to binding agents capable of binding T-cells. The removal of foreign (donor) T-cells from the graft prevents graft vs. host syndrome, which is caused by graft T-cells reactive toward the recipient's MHC antigens. If desired, any of the ex vivo treatments may be followed by removal of cell debris and conjugated enzymes prior to reintroduction, for example by centrifugation or precipitation with anti-immunoglobulin (where antibodies are employed as the binding agents).

The conjugated enzymes of the invention may be prepared substantially in advance, and may be stored in conventional formulations, as is practiced for MAbs in ELISA assay kits. Typically, enzyme-conjugated MAbs are stored in a buffered isotonic solution (in the absence of halide or other enzyme substrates), optionally containing a carrier protein such as human serum albumin. Alternatively, the conjugates may be

EP 0 361 908 A2

lyophilized, usually by freeze-drying a solution of conjugates in the presence of a cryo-protective agent such as HSA, lactose, etc. The conjugates may be provided in the same formulation, or in two separate formulations, as desired. One may provide a convenient kit for treatment, containing suitable quantities of conjugates for testing a sample of cells, an assay dye such as MTT, and other containers holding quantities of conjugates suitable for therapy.

The conjugates of the invention are particularly useful as they provide a very fast, specific killing action, and allow the use of very small amounts. High speed killing is important because it allows the conjugates to kill target cells before the target cells can effect countermeasures, such as spore formation, glutathione up-regulation, shedding of surface antigens, etc.

Further, the combination of conjugated peroxide-generating enzymes with conjugated peroxidase is effective outside the target cell: the conjugates do not need to be brought within the target cell (e.g., by endocytosis) for cytotoxicity, as is the case with most immunotoxins such as ricin. The elimination of endocytosis as a preliminary step to cytotoxicity allows one to use the conjugates of the invention to deactivate free viral particles, as well as the other target cells described above.

Additionally, with the choice of appropriate binding agents, (e.g., IgG$_2$), one may derive additional cytotoxicity through complement fixation.

C. Examples

The examples presented below are provided as a further guide to the practitioner of ordinary skill in the art, and are not to be construed as limiting the invention in any way.

Example 1

(Preparation of Immunotherapeutics)

Conjugated glucose oxidase and myeloperoxidase were prepared as follows:

Glucose oxidase (GO) obtained from Aspergillus niger was purchased from Boehringer Mannheim. MPO was purified from cultured HL-60 cells, as described by P.C. Andrews et al, J Biol Chem (1981) 256:4211-18.

A mouse MAb specific for a B-cell associated antigen was prepared by hyperimmunizing a mouse with human B-cells emulsified in Freund's adjuvant. Hybridomas were prepared following the method of Kohler and Millstein. One clone was selected and designated DLC-48. GO and MPO are each conjugated to purified DLC-48 using glutaraldehyde or other cross-linking agents, as described in the art for general antibody-enzyme conjugation, e.g., by S. Avrameas, Immunochem (1969) 6:43-52. If desired, one may protect the antibody's antigen-binding sites using an anti-Fv MAb, which binds to the antibody variable region. The resulting MAb-GO and MAb-MPO conjugates were repurified to remove non-bound enzyme by size exclusion chromatography on an S-300 column (1.8 x 85 cm) in 10 mM NaOAc, 150 mM NaCl, pH 5.8. Alternatively, the conjugates may be immunoaffinity purified using an anti-Fv MAb-conjugated column, or by using an antigen-conjugated column.

The MAb-GO conjugate may be assayed for enzyme activity by horseradish peroxidase-coupled oxidation of o-dianisidine, following the reaction at 460 nm. A standard curve using purified GO at 460 nm gave about 0.42 OD/min/ug enzyme. The MAb-MPO conjugate may be assayed using H$_2$O$_2$-mediated oxidation of 2,2'-azinobis(3-ethylbenzthiazolinesulfonic acid).

The resulting conjugates may be stored in solution, or preferably lyophilized, using techniques standard in the art for enzyme-coupled antibodies.

Example 2

(In Vitro Cytotoxicity)

7

Cytotoxicity against myeloma cells and lack of toxicity to non-target cells was demonstrated in vitro:

(A) SU4 (human B-cell lymphoma target cells) and SU1 (non-target cells) were plated onto separate 96-well flat-bottom plates at about $1.5 \times 10^5$ cells/well, in a buffer containing $Ca^{++}$ and $Mg^{++}$, and 2% fetal bovine serum (FBS), but lacking halides, glucose, and phenol red. A murine monoclonal antibody specific for SU4 lymphoma cells, DLC-48, was obtained from Dr. Jane Winter (Northwestern U., Chicago, IL), and is described in Int J Cancer (1987) 39:670-77. The first plate received 100 uL/well of DLC-48-conjugated GO (DLC-48/GO) alone at 0.3, 1, 3, 10, and 30 ug/mL. A second plate received 100 uL/well of DLC-48/MPO alone at 0.3, 1, 3, 10, and 30 ug/mL. A third plate received 100 uL/well of DLC-48/GO (0.3 ug/mL) and 100 uL/well of DLC-48/MPO at 0.3, 1, 3, 10, and 30 ug/mL. The plates were then incubated for about 1 hour at 37°C under 5-7% $CO_2$. The plates were washed three times by spinning for 5 minutes at 1200 rpm, removing the liquid, and refilling with buffer. The enzyme substrate was then added (buffer containing 4.5 g/L glucose with 0.1 mM NaI), and the plates incubated for 2 hours at 37°C (5-7% $CO_2$) followed by washing three times as before. The cells were resuspended in 100 uL of RPMI containing 5% FBS, 10 uL/well MTT (5 mg/mL) added, and the plates allowed to incubate for 4 hours. The plates were spun again, and liquid removed. DMSO (100%) was then added to each well (100 uL/well), and absorbance read at 550 nm.

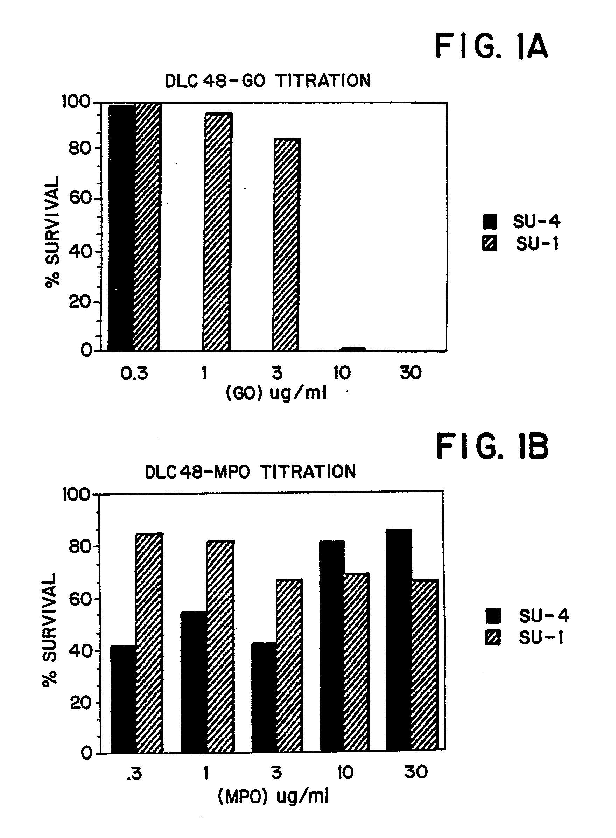

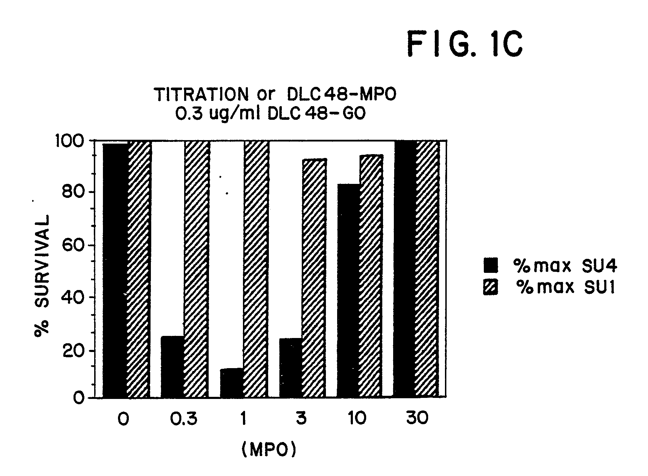

The results are shown in Figure 1. As shown in Fig. 1(A), DLC-48/GO is essentially non-toxic to both target and non-target cells at 0.3 ug/mL, is highly toxic to SU4 target cells and slightly toxic to SU1 cells at concentrations of 1 ug/mL and higher, and is highly toxic to SU1 non-target cells at concentrations of 10 ug mL and higher. As shown in Fig. 1(B), DLC-48/MPO was only moderately toxic to both SU4 and SU1 cells at all concentrations. Figure 1(C) shows that the combination of GO and MPO is highly toxic to SU4 cells at 0.3-3 ug/mL MPO + 0.3 ug/mL GO, and is at most slightly toxic to SU1 cells (at 3-10 ug/mL MPO). This demonstrates that the combination of DLC-48/GO with DLC-48/MPO synergistically kills myeloma cells without damaging non-target cells, at enzyme concentrations which would otherwise either damage non-target cells, or which would fail to provide sufficient target cell killing.

(B) The effect of DLC-48/GO and DLC-48/MPO combinations was further demonstrated through the following clonogenicity assay. This assay achieves sensitivity of 6 log units (detecting 0.0001% of viable cells), and measures the proliferative potential of treated tumor cells.

SU4 cells were suspended at $0.6 \times 10^6$ cells/mL, and were incubated for 2-3 hours at 37°C with 1.2 ug/mL DLC-48/GO and 0.12, 0.25, or 0.50 ug/mL DLC-48/MPO in complete medium ("CM": EMEM with 1% fetal bovine serum, 0.2 mM NaI, and 9.0 g/L D-glucose). The cells were then washed twice, suspended in DMEM-HS 10%, and seeded at $10^4$, $10^3$, $10^2$, 5, 2, and 0.5 cells/well in flat bottomed 96-well NUNC microculture plates (32 replicate wells per seeded concentration) at 100 uL/well. The plates were then placed at 37°C in a $CO_2$ humidified incubator. At day 6, the cells were supplemented with 100 uL of fresh medium, and at day 12 100 uL of medium was removed and replaced with 100 uL of fresh medium. On day 14, the plates were scored for growth by microscopic observation, and the number of wells per 32 at each seeding density showing growth counted. Control wells were treated identically, but received GO and MPO conjugated to monoclonal antibodies which were not reactive with the target SU4 cells. The results are presented in Table 1 below, showing the number of wells/density having growth, and the calculated frequency of surviving cells.

TABLE I

| Clonogenicity Assay | | | | | | |
|---|---|---|---|---|---|---|
| | Cells seeded per well | | | | | |
| DLC-48/MPO* (ug/mL) | $10^4$ | $10^3$ | $10^2$ | 5 | 2 | 0.5 | Freq |
| Control | - | - | - | 23 | 18 | 6 | 1/2.4 |
| Control | - | - | - | 26 | 18 | 7 | 1/2.4 |
| 0.12 | 0 | 6 | 0 | - | - | - | <1/10$^6$ |
| 0.25 | 0 | 0 | 0 | - | - | - | <1/10$^6$ |
| 0.50 | 4 | 3 | 0 | - | - | - | <1/10$^6$ |

* Each non-control well also received 1.2 ug/mL DLC-48/GO

Example 3

(Inhibition of Viruses)

This experiment demonstrates the direct deactivation of viral particles (exemplified herein by HSV-2) using the conjugates of the invention.

Using the procedure set forth in Example 1, GO and hMPO were conjugated to anti-HSV-2 MAbs designated "MPI", which are described in J Virol (1988) 62:4027-36 and J Virol (1985) 53:634-44 (obtained from Dr. R. Eisenberg, Department of Medicine, U. Pennsylvania, Philadelphia, PA). The conjugates are referred to herein as "GO-MAb" and "MPO-MAb" respectively. Herpes simplex virus 2 (HSV-2) strain MS seed stock, having a titer of $2 \times 10^5$ PFU/mL, was obtained from the American Type Culture Collection (ATCC), accession no. VRS40. The seed stock was thawed and diluted 1:1 with 2X solutions of GO-MAb and MPO-MAb in complete medium ("CM") to provide GO-MAb:MPO-MAb concentrations of 22:0 ug/mL, 0:86 ug/mL, 2:9 ug/mL, 2:86 ug/mL, 22:9 ug/mL, and 22:86 ug/mL. The mixtures were incubated at 37°C for one hour.

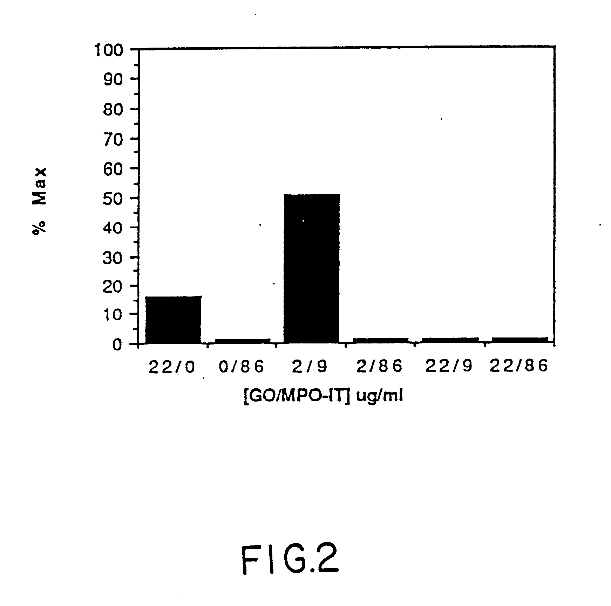

After incubation, the mixture was diluted 1:500, and 0.2 mL inoculated into 35 mm, 24-hour old VERO cell (ATCC CRL1587) monolayers. Virus was allowed to adsorb for 1 hour at 37°C. After adsorption, the cells were overlaid with 1% agarose, and incubated for 3-5 days at 37°C, until plaque formation was complete. The monolayers were stained with 1% neutral red for one hour, destained in PBS for 2-3 hours, and photographed. The results are graphically depicted in Figure 2, as the percentage reduction of plaques from control. As shown in the figure, GO-MAb alone at 22 ug/mL was able to reduce plaque formation by about 83%, and MPO-MAb at 86 ug/mL effected substantially complete inhibition. The combination of GO-MAb and MPO-MAb at 2 and 9 ug/mL respectively inhibited plaque formation by about 50%, while combinations of 2 ug/mL GO-MAb and 86 mg/mL MPO-MAb, 22 ug/mL GO-MAb with 86 ug/mL MPO-MAb, and 22 ug/mL GO-MAb with 9 ug/mL MPO-MAb effected substantially complete viral inhibition.

Example 4

(Inhibition of Fungi)

Inhibition of fungal growth (exemplified by Candida albicans) was demonstrated using GO and MPO conjugated to a murine anti-C. albicans MAb denoted 9/14/35 ("MAb") obtained from Chemunex S.A.,

Maisons Alfort, France (other anti-C. albicans MAbs may be prepared by immunizing mice with C. albicans using standard methods, and following Kohler and Millstein, Nature (1975) 256:495-96). The conjugates were prepared as described in Example 1 above.

A 1.0 OD$_{600}$ culture of C. albicans (100 uL) was diluted 1:100 in Saboraud's dextrose broth (SDB) and incubated with the GO-MAb and MPO-MAb conjugates at 37°C for 24 hours in 96 well plates. After 24 hours, the wells were visually scored for presence or absence of growth. The results are reported below in Table 2. The reported conjugate concentrations are based on enzyme activity, i.e., "8 ug/mL GO-MAb" corresponds to an amount of GO-MAb having the same activity as free GO at 8 ug/mL.

TABLE II

| MPO-MAb (ug/mL) | Growth ( + -) | | |
| --- | --- | --- | --- |
| | (16 ug/mL) | GO-MAb | (8 ug/mL) |
| 21 | - | | - |
| 10.5 | - | | - |
| 5.25 | - | | - |
| 2.62 | - | | + |
| 1.31 | - | | + |
| 0.66 | - | | + |
| 0.33 | + | | + |
| 0.00 | + | | + |

As shown in Table 2, 16 ug/mL of GO-MAb with 0.66 ug/mL MPO-MAb or 8 ug/mL GO-MAb with 5.25 ug/mL MPO-MAb is sufficient to inhibit C. albicans growth, even in the absence of added iodide.

Example 5

(Formulations)

(A) A formulation of the invention is prepared as follows:

| DLC-48/GO | 0.5 ug |
| --- | --- |
| DLC-48/MPO | 1.0 ug |
| HSA | 100.0 mg |
| Acetate buffer | 100.0 mM |
| Sterile water for injection qs | 1.0 mL |

The components are mixed, sterile filtered, and packaged.
(B) A lyophilized formulation is prepared by freeze-drying the formulation of part A above.

Claims

1. A composition for killing a target cell within a population of desired cells without killing said desired cells, wherein said target cell is characterized by a surface antigen not found on said desired cells, wherein said composition comprises:
an effective amount of peroxidase selected from myeloperoxidase, eosinophil peroxidase, thyroperoxidase, and salivary peroxidase, wherein said peroxidase is chemically conjugated to a first binding agent capable of specifically binding to said surface antigen; and

a sufficient amount of a hydrogen peroxide-producing enzyme chemically conjugated to a second binding agent capable of specifically binding to said surface antigen.

2. A composition according to claim 1 wherein said hydrogen peroxide-producing enzyme is glucose oxidase, NADPH oxidase, amino acid oxidase, alcohol oxidase, xanthine oxidase, cholesterol oxidase, or choline oxidase.

3. A composition according to claim 1 or 2 wherein said first and second binding agents independently comprise antibodies or antigen-binding portions of antibodies.

4. A composition according to any one of the preceding claims wherein said peroxidase and said hydrogen peroxide-producing enzyme are conjugated to separate but identical binding agents.

5. A composition according to any one of claims 1 to 3 wherein said peroxidase and said hydrogen proxide-producing enzyme are conjugated to the same binding agent to form a peroxidase-binding agent-enzyme conjugate.

6. A method for preparing bone marrow for bone marrow transplant by selectively removing undesirable target cells distinguishable from desired bone marrow cells by a surface antigen, which method comprises: contacting said bone marrow in vitro with a composition according to any one of the preceding claims.

7. A method according to claim 6 wherein said target cells are T-cells, and said surface antigen comprises a protein characteristic of T-cells.

8. A method according to claim 7 wherein said surface antigen is T4 antigen, T8 antigen, or Thy1 antigen.

9. A method according to any one of claims 6 to 8 wherein said target cells are B-cell lymphoma cells.

10. A composition according to any one of claims 1 to 5 for use in a method of treating the human or animal body for killing target cells bearing a surface antigen characteristic of said target cells in a subject having such cells.

11. A composition according to claim 10 for use in a method which further comprises administering a sufficient quantity of substrate for said hydrogen peroxide-producing enzyme to provide a sufficient amount of hydrogen peroxide for cytotoxicity.

12. A composition according to claim 10 or 11 for use in a method wherein said target cells are tumour cells, and said surface antigen comprises a tumour-specific antigen.

13. A composition according to claim 10 or 11 for use in a method wherein said target cells are autoimmune T-cells bearing T-cell antigen receptors, or B-lymphocytes bearing surface-bound immunoglobulin, and said first and second binding agents comprise ligands for said T-cell antigen receptors or surface-bound immunoglobulin.

14. A composition according to claim 10 or 11 for use in a method wherein said target cells are cells infected with virus, and said surface antigen comprises a viral antigen.

15. A composition according to claim 10 or 11 for use in a method wherein said target cells are bacteria, fungi, yeast, or viral particles.

# FIG. IA

## DLC 48-GO TITRATION

# FIG. IB

## DLC 48-MPO TITRATION

# FIG. 1C

TITRATION or DLC 48-MPO
0.3 ug/ml DLC 48-GO

■ %max SU4
▨ %max SU1

FIG.2